# FASCICULE DE BREVET EUROPEEN

(11) **EP 1 614 687 B1**
(45) Date de publication et mention de la délivrance du brevet: **11.11.2009**
(21) Numéro de dépôt: 05290383.8
(22) Date de dépôt: 21.02.2005
(51) Int. Cl.: C07D 405/06

(54) **Nouveau procédé de synthèse de dérivés de la 1,3,4,5-tétrahydro-2H-3-benzazépin-2-one, et application à la synthèse de l'ivabradine et de ses sels d'addition à un acide pharmaceutiquement acceptable**
Verfahren zur Herstellung von 1,3,4,5-tetrahydro-2H-3-Benzazepin-2-on-Derivaten und dessen Anwendung in der Herstellung von Ivabradin sowie dessen pharmazeutisch verträglichen Salze
Process for the preparation of 1,3,4,5-tetrahydro-2H-3-benzazepin-2-one derivatives and their application in the synthesis of ivabradine and its pharmaceutically acceptable salts

(30) Priorité: 13.04.2004 FR 0403828
(43) Date de publication de la demande: 11.01.2006
(73) Titulaire: Les Laboratoires Servier, 92415 Courbevoie Cédex (FR)
(72) Inventeur: Lerestif, Jean-Michel, 76190 Yvetot (FR); Lecouve, Jean-Pierre, 76600 Le Havre (FR); Souvie, Jean-Claude, 76600 Le Havre (FR); Brigot, Daniel, 76190 Sainte-Marie-Des-Champs (FR)

(56) Documents cités:
- EP-A- 0 204 349
- EP-A- 0 534 859
- EP-A- 1 589 005

## Description

La présente invention concerne un procédé de synthèse de dérivés de la 1,3,4,5-tétrahydro-2*H*-3-benzazépin-2-one, et leur application à la synthèse de l'ivabradine et de ses sels d'addition à un acide pharmaceutiquement acceptable.

Plus spécifiquement, la présente invention concerne un procédé de synthèse des composés de formule (I) : 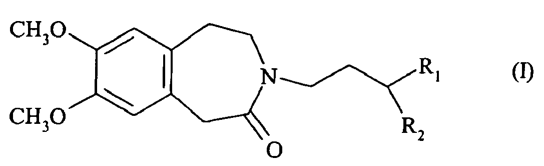 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane.

Les composés de formule (I) obtenus selon le procédé de l'invention sont utiles dans la synthèse de l'ivabradine de formule (II) : 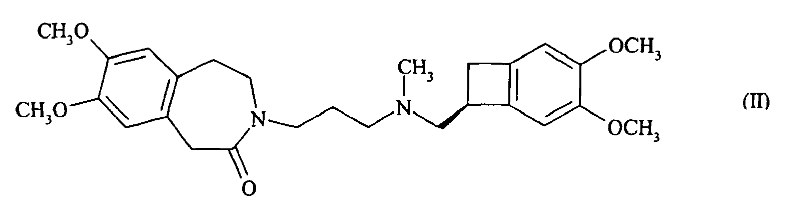 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de ses hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Ce brevet décrit la synthèse du chlorhydrate de l'ivabradine par réaction du composé de formule (III) : 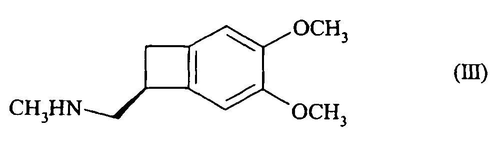 avec le composé de formule (IV) : 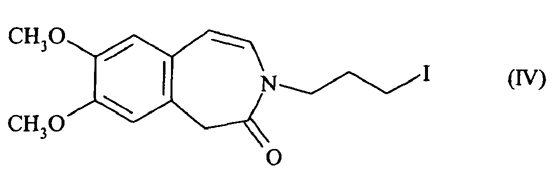 pour conduire au composé de formule (V) : 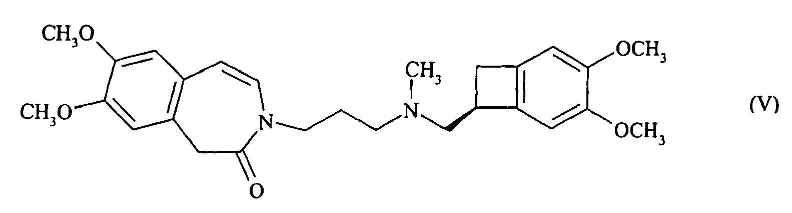 dont l'hydrogénation catalytique conduit à l'ivabradine, qui est alors transformée en son chlorhydrate.

Cette méthode a l'inconvénient de ne conduire au chlorhydrate de l'ivabradine qu'avec un rendement très faible, inférieur à 17 % sur l'ensemble des 3 étapes.

Ce rendement très faible est dû en partie au rendement médiocre de l'étape d'hydrogénation catalytique de la fonction 1,3-dihydro-2*H*-3-benzazépin-2-one du composé de formule (V) en 1 ,3,4,5-tétrahydro-2*H*-3-benzazépin-2-one correspondante.

En effet, dans les conditions utilisées (hydrogénation catalysée par l'hydroxyde de palladium à 10%, à température ambiante, dans l'acide acétique glacial), le rendement de cette réaction de réduction n'est que de 40%.

Or, compte-tenu de l'intérêt pharmaceutique de l'ivabradine et de ses sels, il était impératif de pouvoir accéder au dérivé de 1,3,4,5-tétrahydro-2*H*-3-benzazépin-2-one de formule (I) avec un procédé industriel performant, et notamment avec un bon rendement.

Au vu du rendement médiocre décrit dans EP 0534859 pour la réduction de la fonction 1,3-dihydro-2*H*-3-benzazépin-2-one, une hydrogénation catalytique ne semblait pas pouvoir remplir un tel cahier des charges.

La Demanderesse a pourtant trouvé que, de manière surprenante, le choix de conditions réactionnelles bien spécifiques, en particulier du solvant, permettait d'accéder au dérivé de 1,3,4,5-tétrahydro-2*H*-3-benzazépin-2-one de formule (I) par hydrogénation catalytique de la 1,3-dihydro-2*H*-3-benzézapin-2-one correspondante, avec un très bon rendement.

Plus spécifiquement, la présente invention concerne un procédé de synthèse des composés de formule (I) : 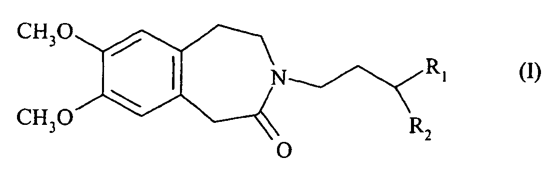 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
caractérisé en ce que l'on soumet le composé de formule (VI) : 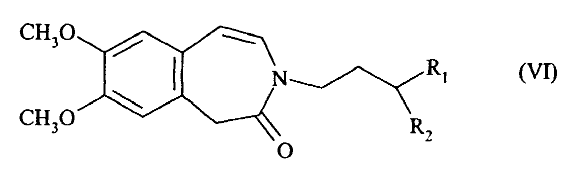 dans laquelle R₁ et R₂ sont tels que définis précédemment,
à une réaction d'hydrogénation catalytique,
dans un solvant non acide,
puis que l'on filtre le milieu réactionnel,
pour conduire au composé de formule (I).

Parmi les solvants non acides utilisables pour le procédé de l'invention, on citera à titre non limitatif les acétates, les alcools, préférentiellement l'éthanol, le méthanol ou l'isopropanol, le tétrahydrofurane, le toluène, le dichlorométhane et le xylène.

Parmi les catalyseurs utilisables pour le procédé de l'invention, on peut citer à titre non limitatif le palladium, le platine, le nickel, le ruthénium, le rhodium, ainsi que leurs dérivés, notamment sous forme supportée ou sous forme d'oxydes.
Le catalyseur préféré est le palladium sur charbon.

La température de la réaction d'hydrogénation du composé de formule (VI) est préférentiellement comprise entre 20 et 100°C, plus préférentiellement entre 40°C et 80°C, encore plus préférentiellement entre 45 et 65°C.

La pression d'hydrogène lors de la réaction d'hydrogénation du composé de formule (VI) est préférentiellement comprise entre 1 et 220 bars, plus préférentiellement entre 1 et 100 bars, encore plus préférentiellement entre 1 et 30 bars.

Dans le procédé selon l'invention, les composés de formule (VI) préférentiellement utilisés sont les composés de formule (VIa), cas particuliers des composés de formule (VI) pour lesquels R₁ et R₂ forment ensemble avec l'atome de carbone qui les porte un cycle 1,3-dioxane, 1,3-dioxolane ou 1,3-dioxépane.

Les composés de formule (I) sont des produits utiles comme intermédiaires de synthèse dans l'industrie chimique ou pharmaceutique, notamment dans la synthèse de l'ivabradine et de ses sels d'addition à un acide pharmaceutiquement acceptable.

A titre d'exemple, la déprotection du diacétal de formule (I) conduit à l'aldéhyde de formule (VII) : 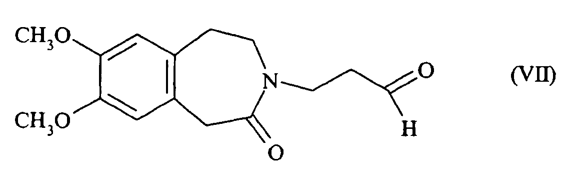 qui est mis en réaction avec la (7S)-3,4-diméthoxybicyclo[4.2.0]octa-1,3,5-trién-7-yl]-N-méthylméthanamine dans des conditions d'amination réductrice, pour conduire à l'ivabradine.

Les composés de formule (I) préférés sont ceux pour lesquels R₁ et R₂ forment ensemble avec l'atome de carbone qui les porte un cycle 1,3-dioxane, 1,3-dioxolane ou 1,3-dioxépane.

L'exemple ci-dessous illustre l'invention.

### EXEMPLE : 3-[2-(1,3-Dioxolan-2-yl)-éthyl]-7,8-diméthoxy-1,3,4,5-tétrabydro-2H-3-benzazépin-2-one

Dans un hydrogénateur, charger 100 g de 3-[2-(1,3-dioxolan-2-yl)-éthyl]-7,8-diméthoxy-1,3-dihydro-2*H*-3-benzazépin-2-one, 500 ml d'isopropanol et 10 g de Pd/C. Purger à l'azote puis à l'hydrogène, chauffer à 60°C, puis hydrogéner à cette température sous une pression de 1 bar pendant 4h.
Filtrer le milieu réactionnel à 60°C afin d'éliminer le catalyseur.
Rincer avec 2 x 50 ml d'isopropanol.
Refroidir à 50°C et ajouter 200 ml de tert-butyl méthyl éther (MTBE).
Refroidir à 20°C puis glacer à 5°C pendant 1h00.
Filtrer les cristaux obtenus à 5°C. Sécher jusqu'à poids constant.
Le produit attendu est obtenu avec un rendement de 88 %, et une pureté chimique supérieure à 98 %.

## Revendications

1. Procédé de synthèse des composés de formule (I) : 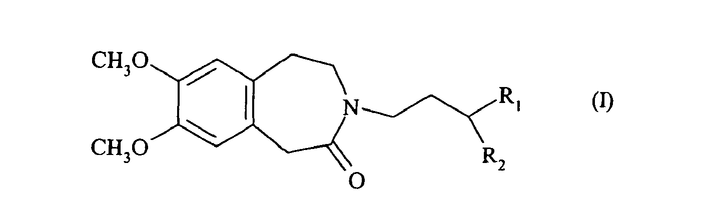 dans laquelle R₁ et R₂, identiques ou différents, représentent chacun un groupement alkoxy (C₁-C₈) linéaire ou ramifié, ou bien forment ensemble avec l'atome de carbone qui les porte un cycle 1,3-dioxane, 1,3-dioxolane ou 1,3-dioxépane,
**caractérisé en ce que** l'on soumet le composé de formule (VI) : 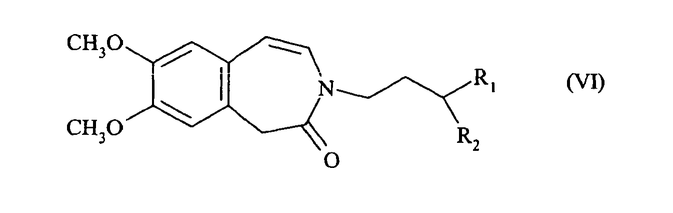 dans laquelle R₁ et R₂ sont tels que définis précédemment,
à une réaction d'hydrogénation catalytique,
dans un solvant non acide,
puis que l'on filtre le milieu réactionnel,
pour conduire au composé de formule (I).

2. Procédé de synthèse selon la revendication 1, **caractérisé en ce que** le catalyseur de la réaction d'hydrogénation du composé de formule (VI) est le palladium sur charbon.

3. Procédé de synthèse selon l'une quelconque des revendications 1 ou 2, **caractérisé en ce que** la pression d'hydrogène lors de la réaction d'hydrogénation du composé de formule (VI) est comprise entre 1 et 220 bars.

4. Procédé de synthèse selon l'une quelconque des revendications 1 à 3, **caractérisé en ce que** la réaction d'hydrogénation du composé de formule (VI) est conduite dans un solvant alcoolique.

5. Procédé de synthèse selon la revendication 4, **caractérisé en ce que** le solvant alcoolique est l'éthanol, le méthanol ou l'isopropanol.

6. Procédé de synthèse selon l'une quelconque des revendications 1 à 5, **caractérisé en ce que** la température est comprise entre 20 et 100°C.

7. Procédé de synthèse selon la revendication 6, **caractérisé en ce que** la température est comprise entre 40 et 80°C.

8. Procédé de synthèse selon l'une quelconque des revendications 1 à 7, **caractérisé en ce qu'**il utilise comme matière première un composé de formule (Vla), cas particulier des composés de formule (VI) pour lesquels R₁ et R₂ forment ensemble avec l'atome de carbone qui les porte un cycle 1,3-dioxane, 1,3-doxolane ou 1,3-dioxépane.

9. Procédé de synthèse de l'ivabradine, de ses sels pharmaceutiquement acceptables et de ses hydrates, dans lequel le composé de formule (VI) est transformé en intermédiaire de formule (I) suivant le procédé de la revendication 1, puis l'intermédiaire de formule (I) est transformé en ivabradine.

## Claims

1. Process for the synthesis of compounds of formula (I) : 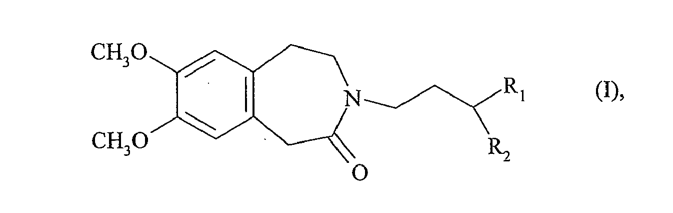 wherein R₁ and R₂, which may be the same or different, each represent a linear or branched (C₁-C₈)alkoxy group or form, together with the carbon atom carrying them, a 1,3-dioxane, 1,3-dioxolane or 1,3-dioxepane ring,
**characterised in that** the compound of formula (VI) : 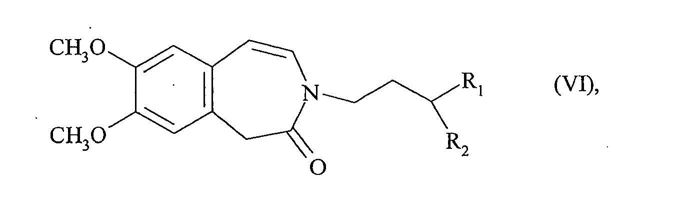 wherein R₁ and R₂ are as defined hereinbefore,
is subjected to a catalytic hydrogenation reaction,
in a non-acid solvent,
and then the reaction mixture is filtered
to yield the compound of formula (I).

2. Synthesis process according to claim 1, **characterised in that** the catalyst for the hydrogenation reaction of the compound of formula (VI) is palladium-on-carbon.

3. Synthesis process according to either claim 1 or claim 2, **characterised in that** the hydrogen pressure during the hydrogenation reaction of the compound of formula (VI) is from 1 to 220 bars.

4. Synthesis process according to any one of claims 1 to 3, **characterised in that** the hydrogenation reaction of the compound of formula (VI) is carried out in an alcoholic solvent.

5. Synthesis process according to claim 4, **characterised in that** the alcoholic solvent is ethanol, methanol or isopropanol.

6. Synthesis process according to any one of claims 1 to 5, **characterised in that** the temperature is from 20 to 100°C.

7. Synthesis process according to claim 6, **characterised in that** the temperature is from 40 to 80°C.

8. Synthesis process according to any one of claims 1 to 7, **characterised in that** it uses, as starting material, a compound of formula (VIa), a particular case of the compounds of formula (VI) wherein R₁ and R₂ form, together with the carbon atom carrying them, a 1,3-dioxane, 1,3-dioxolane or 1,3-dioxepane ring.

9. Process for the synthesis of ivabradine, pharmaceutically acceptable salts thereof, and hydrates thereof, wherein the compound of formula (VI) is converted into the intermediate of formula (I) in accordance with the process of claim 1, and then the intermediate of formula (I) is converted into ivabradine.

## Patentansprüche

1. Verfahren zur Synthese der Verbindungen der Formel (I): 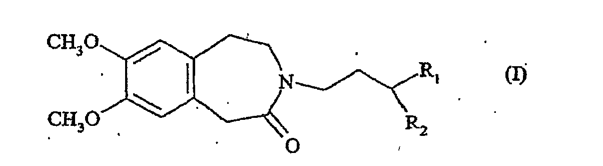 in der R₁ und R₂, die identisch oder verschieden sind, jeweils eine geradkettige oder verzweigte (C₁-C₈)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden,
**dadurch gekennzeichnet, dass** man die Verbindung der Formel (VI): 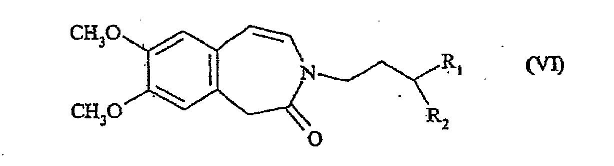 in der R₁ und R₂ die oben angegebenen Bedeutungen besitzen,
einer katalytischen Hydrierreaktion
in einem nicht sauren Lösungsmittel unterwirft
und dann das Reaktionsmedium filtriert
zur Gewinnung der Verbindung der Formel (I).

2. Syntheseverfahren nach Anspruch 1, **dadurch gekennzeichnet, dass** der Katalysator für die Reaktion der Hydrierung der Verbindung der Formel (VI) Palladium-auf-Kohlenstoff ist.

3. Syntheseverfahren nach einem der Ansprüche 1 oder 2, **dadurch gekennzeichnet, dass** der Wasserstoffdruck bei der Reaktion der Hydrierung der Verbindung der Formel (VI) zwischen 1 und 220 bar liegt.

4. Syntheseverfahren nach einem der Ansprüche 1 bis 3, **dadurch gekennzeichnet, dass** die Reaktion der Hydrierung der Verbindung der Formel (VI) in einem alkoholischen Lösungsmittel durchgeführt wird.

5. Syntheseverfahren nach Anspruch 4, **dadurch gekennzeichnet, dass** das alkoholische Lösungsmittel Ethanol, Methanol oder Isopropanol ist.

6. Syntheseverfahren nach einem der Ansprüche 1 bis 5, **dadurch gekennzeichnet, dass** die Temperatur zwischen 20 und 100 °C liegt.

7. Syntheseverfahren nach Anspruch 6, **dadurch gekennzeichnet, dass** die Temperatur zwischen 40 und 80 °C liegt.

8. Syntheseverfahren nach einem der Ansprüche 1 bis 7, **dadurch gekennzeichnet, dass** man als Ausgangsmaterial eine Verbindung der Formel (VIa) verwendet, einem Sonderfall der Verbindungen der Formel (VI), worin R₁ und R₂ gemeinsam mit dem sie tragenden Kohlenstoffatom einen 1,3-Dioxan-, 1,3-Dioxolan- oder 1,3-Dioxepan-Ring bilden.

9. Verfahren zur Synthese von Ivabradin, seinen pharmazeutisch annehmbaren Salzen und seinen Hydraten, worin die Verbindung der Formel (VI) nach dem Verfahren von Anspruch 1 in das Zwischenprodukt der Formel (I) umgewandelt wird, wonach das Zwischenprodukt der Formel (I) in Ivabradin umgewandelt wird.
